# EUROPEAN PATENT APPLICATION

(11) **EP 4 414 444 A1**
(43) Date of publication of application: **14.08.2024**
(21) Application number: 22878746.1
(22) Date of filing: 15.09.2022
(51) Int. Cl.: C12M 1/00, C12M 3/00, C12M 1/12, C12M 1/107

(54) **BIOREACTOR FOR SUSPENDED CELLS**

(30) Priority: 05.10.2021 KR 20210131902
(71) Applicant: Amogreentech Co., Ltd., Gimpo-si, Gyeonggi-do 10014 (KR)
(72) Inventor: JANG, Seon Ho, Gimpo-si, Gyeonggi-do 10014 (KR); HAN, Kyoung Ku, Gimpo-si, Gyeonggi-do 10014 (KR); SEO, In Yong, Gimpo-si, Gyeonggi-do 10014 (KR); LEE, Seoung Hoon, Gimpo-si, Gyeonggi-do 10014 (KR); KOO, Song Hee, Gimpo-si, Gyeonggi-do 10014 (KR); NOH, Hyeong Tak, Gimpo-si, Gyeonggi-do 10014 (KR); KIM, Ji Young, Gimpo-si, Gyeonggi-do 10014 (KR); LEE, Su Yeon, Gimpo-si, Gyeonggi-do 10014 (KR)
(74) Representative: Treeby, Philip David William
(86) International application number: PCT/KR2022/013821
(87) International publication number: WO 2023/058925

(57) **Abstract**

A bioreactor for suspended cells is provided. A bioreactor for suspended cells, according to one embodiment of the present invention, comprises: a housing which is formed in the shape of a container having a culture space with a predetermined volume, and which comprises pots for seeding; a support assembly comprising a plurality of supports arranged at intervals in multiple stages in one direction of the housing; at least one gas inlet penetrating the housing by a predetermined area; and a porous member for covering the gas inlet, wherein the culture space comprises a medium storage space filled with a predetermined amount of a medium so that all the plurality of supports are submerged therein, and the medium storage space can comprise a plurality of cell growth spaces divided through the plurality of supports arranged at intervals in multiple stages.

## Description

### [Technical field]

The present invention relates to a bioreactor for suspended cells.

### [Background Art]

Bioprocess refers to the process of producing a desired therapeutic agent using living cells in the bio field.

Antibodies, stem cells, and immune cells are produced through cell culture and used to produce biopharmaceuticals, vaccines, and cell therapeutic agents.

Depending on their ability to adhere, cells are classified into adherent cells that must adhere to the surface substrate and suspended cells that proliferate without adherence to the substrate surface.

In other words, adherent cells are cultured while attached to a support that serves as a surface substrate, whereas suspended cells are not cultured while attached to the support, but grow by receiving signals while repeating the process of contact-suspension-contact-suspension, such as attaching and falling off the surface of the support, and then attaching and falling off the surface of the support again.

Among these suspended cells, T cells included in white blood cells are cultured for about 14 days after adding both a single-layer support with a predetermined area and a predetermined volume of liquid medium into a backpack formed of a gas-permeable film with a predetermined volume.

Accordingly, when culturing T cells, since the nutrients included in the medium are consumed over time along with the growth of the cells, a new medium needs to be added to the backpack a week after the start of the culture.

However, in the conventional culture method using a backpack, since the interior of the backpack is formed as one space, the medium including a single layer of support and suspended cells cannot help but be disposed together in the one same space. Accordingly, there is a problem in that each suspended cell included in the medium cannot grow smoothly due to the low frequency of contact with the support.

In addition, in the conventional culture method using a backpack, a new medium can be added to the inside of the backpack, but since the medium cannot be replaced, even when a new medium is added, it has no choice but to be mixed with the existing medium, therefore, there is a problem that the amount of nutrients contained in the medium per unit volume is insufficient.

### [Disclosure]

### [Technical Problem]

The present invention was conceived in consideration of the above points, and the purpose is to provide a bioreactor for suspended cells that may increase the frequency of contact between suspended cells and the support even when supports having the same total area are used by dividing the medium-filled space into a plurality of spaces by arranging a plurality of supports in multiple stages.

Another object of the present invention is to provide a bioreactor for suspended cells in which the added medium and the existing medium can be smoothly mixed when adding the medium.

In addition, still another object of the present invention is to provide a bioreactor for suspended cells that may replace all or part of the existing medium.

### [Technical Solution]

In order to solve the above-described problem, the present invention provides a bioreactor for suspended cells including a housing formed in a box shape having a culture space with a predetermined volume and including a seeding port for seeding suspended cells into the culture space through a medium containing the suspended cells on one side; a support assembly formed of a plate-shaped member having a predetermined area and including a plurality of supports disposed in multiple stages at intervals along one direction of the housing; a gas inlet formed through the housing by a predetermined area to allow gas to flow into the culture space from the outside; and a porous member covering the gas inlet to allow the gas to enter the culture space from the outside while preventing the medium from leaking to the outside, wherein the culture space includes a medium storage space filled with a certain amount of medium so that all of the plurality of supports are submerged and a gas storage space located above the medium storage space and filled with gas, and the medium storage space includes a plurality of cell growth spaces separated from each other by the plurality of supports disposed in multiple stages at intervals.

In addition, the support may include a plate-shaped support member having a predetermined area, and a pair of nanofiber membranes respectively attached to both sides of the support member via an adhesive layer, and the nanofiber membrane may be a motif-coated plate-shaped nanofiber membrane.

In addition, the support may include a plurality of through holes formed through the support member to allow gas introduced into the gas storage space from the outside to pass smoothly.

In addition, the support assembly may include upper and lower plates with a predetermined area, a plurality of supports disposed between the upper and lower plates so that one surfaces face each other, a spacing member disposed between two supports facing each other so as to separate the two supports, and a plurality of fastening bars that fasten and integrate the upper plate, the lower plate, the plurality of supports, and the spacing member.

In addition, the housing may include a housing body in a box shape having a culture space with an open upper portion and a cover covering the open upper portion of the housing body, and at least one fastening bar among the plurality of fastening bars may be disposed in the culture space so that a lower end thereof comes into contact with a bottom surface of the culture space and an upper end thereof comes into contact with an inner surface of the cover to prevent the support assembly from floating in the culture space.

In addition, fixing grooves that are recessed to a certain depth may be respectively formed in an inner surface of the housing body and the inner surface of the cover so that both ends of the fastening bar are inserted and fixed to a certain depth.

In addition, the bioreactor for suspended cells may include at least one blocking member fastened to a side of the support assembly to cover an open side of the cell growth space.

In addition, the support assembly may be disposed in the medium storage space to be spaced a certain distance from an inner surface of the housing where the seeding port is formed, the medium storage space may include a first space in which the support assembly is disposed and a second space formed between the support assembly and the inner surface of the housing where the seeding port is formed, and the first space may be divided into the plurality of cell growth spaces through the plurality of supports.

In addition, the porous member may be a water-repellent membrane.

In addition, the bioreactor for suspended cells may further include a vent hole formed in the housing to communicate with the gas storage space and a port formed in the housing to supply a medium to the culture space or discharge the medium present in the culture space to the outside.

In addition, the bioreactor for suspended cells may further include an extension tube connected to the port in the culture space and having a predetermined length, and a lower end of the extension tube may be disposed in the culture space to be located at a lower position than the support located at the top among the plurality of supports constituting the support assembly.

### [Advantageous Effects]

According to the present invention, by dividing the cell growth space into a plurality of spaces, the frequency of contact between suspended cells floating in the medium and the support can be increased, and thus cell-to-cell interactions can occur more frequently, which can promote the growth of suspended cells.

In addition, according to the present invention, when adding a medium, the added medium and the existing medium can be mixed smoothly so that the nutrients included in the medium per unit volume can be evenly distributed, thereby promoting the growth of suspended cells.

In addition, the present invention can replace the entire existing medium or part of the existing medium, thereby promoting the growth of suspended cells by smoothly supplying the nutrients necessary for the growth of suspended cells.

### [Brief Description of Drawings]

FIG. 1 is a schematic diagram showing a bioreactor for suspended cells according to one embodiment of the present invention.
FIG. 2 is a view in which the main components in FIG. 1 are separated.
FIG. 3 is a cross-sectional view taken in the A-A direction of FIG. 1.
FIG. 4 is a cross-sectional view taken in the B-B direction of FIG. 1, showing a state in which the culture space is filled with a medium.
FIG. 5 is a view showing a modified form of the extension tube in FIG. 4.
FIG. 6 is a view of the support assembly in FIG. 2 taken out and separated.
FIG. 7 is a partial cutaway view showing the detailed layered structure of a support that can be used in a bioreactor for suspended cells according to one embodiment of the present invention.
FIG. 8 is a view showing a bioreactor for suspended cells according to another embodiment of the present invention, with its main components separated.

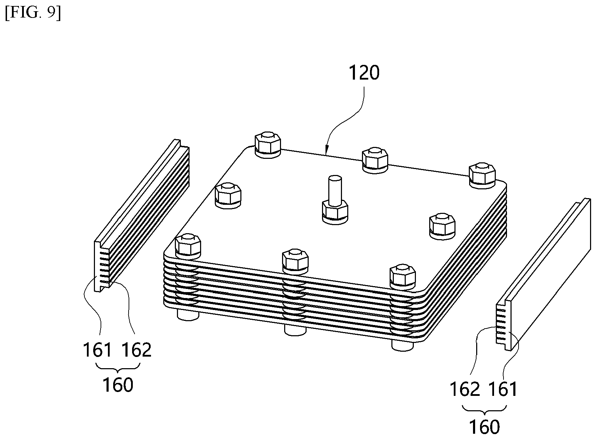

FIG. 9 is a view showing the support assembly and the blocking member in FIG. 8 taken out and separated.
FIG. 10 is a combined view of FIG. 8, showing a state in which the cover is removed.
FIG. 11 is a usage diagram showing the process of seeding suspended cells using a seeding port in a bioreactor for suspended cells according to one embodiment of the present invention.
FIG. 12 is a usage diagram showing the process of supplying a medium to the culture space using a port in a bioreactor for suspended cells according to one embodiment of the present invention.

### [Modes of the Invention]

Hereinafter, embodiments of the present invention will be described in detail with reference to the accompanying drawings so as to be easily carried out by those skilled in the art. The present invention may be embodied in many different forms and is not limited to the embodiments set forth herein. In order to clearly illustrate the present invention in the drawings, portions that are irrelevant to the description are omitted, and the same reference numerals are added to the same or similar elements throughout the specification.

A bioreactor for suspended cells 100 according to one embodiment of the present invention may include a culture space S in which a plurality of supports 121 for cell culture are disposed in multiple stages at predetermined intervals, the culture space S may be divided into a medium storage space S1 filled with a certain amount of medium and a gas storage space S2 filled with gas, and the medium storage space S1 may be divided into a plurality of cell growth spaces 5111 separated from each other by the plurality of supports 121 disposed in multiple stages at intervals.

In the present invention, a medium supplied from the outside may be filled in the culture space S so that all of the plurality of supports 121 may be submerged together with the cell growth space S111 formed between two supports 121 spaced apart from each other at regular intervals. Through this, the culture space S may be divided into the medium storage space S1 and the gas storage space S2.

In addition, the medium may be a medium including suspended cells to be cultured, and the medium may further include magnetic particles coated with a peptide motif in addition to the suspended cells. Accordingly, the suspended cells included in the medium may repeat the process of falling after contacting the two supports 121 that define each cell growth space S111 while floating within each cell growth space S111 formed between the two supports 121, and the suspended cells may grow by receiving signals from the supports 136 and 136' while repeating the process of falling after contacting the support 121.

At this time, in the bioreactor for suspended cells 100 according to one embodiment of the present invention, the medium storage space S1 filled with the medium may be divided into a plurality of cell growth spaces S111 through a plurality of supports 121 disposed in multiple stages at intervals as described above.

Accordingly, in the bioreactor for suspended cells 100 according to one embodiment of the present invention, since one medium storage space S1 filled with the medium may be divided into a plurality of cell growth spaces S111 where cells may grow, the frequency of contact between the suspended cells floating in the medium and the support 121 may be increased. Through this, in the bioreactor for suspended cells 100 according to one embodiment of the present invention, cell-to-cell interactions can occur more frequently in a confined cell growth space S111 defined by two supports 121, which may promote the growth of suspended cells.

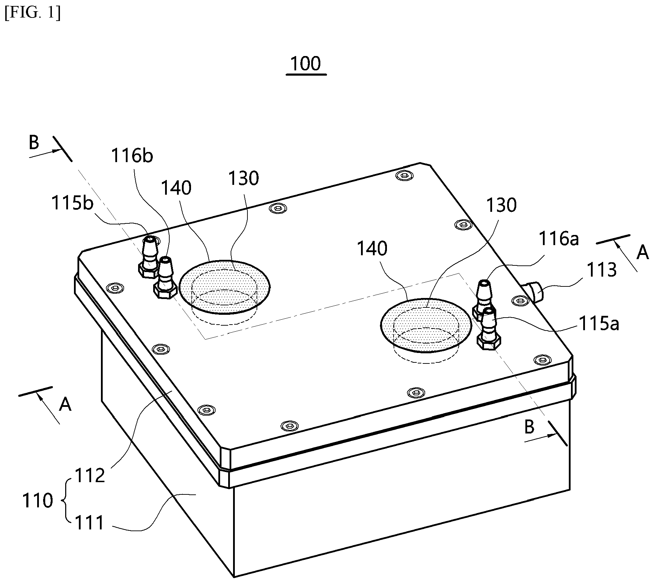

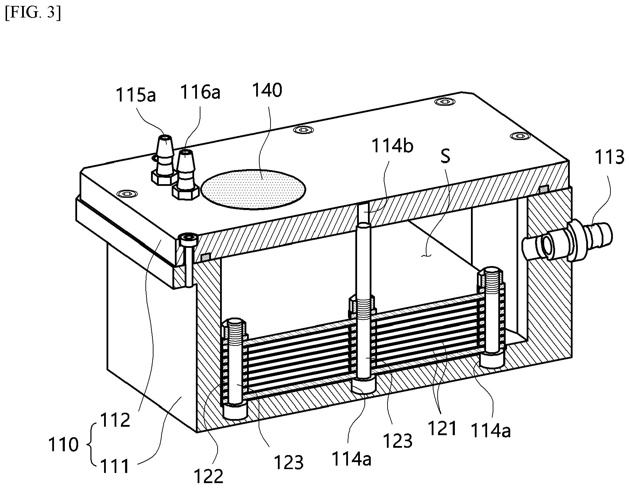

To this end, the bioreactor for suspended cells 100 according to one embodiment of the present invention includes a housing 110, a support assembly 120, a gas inlet 130, and a porous member 140 as shown in FIGS. 1 to 3.

The housing 110 may accommodate the support assembly 120 and a medium for cell culture therein. To this end, the housing 110 may be formed in a box shape with a culture space S.

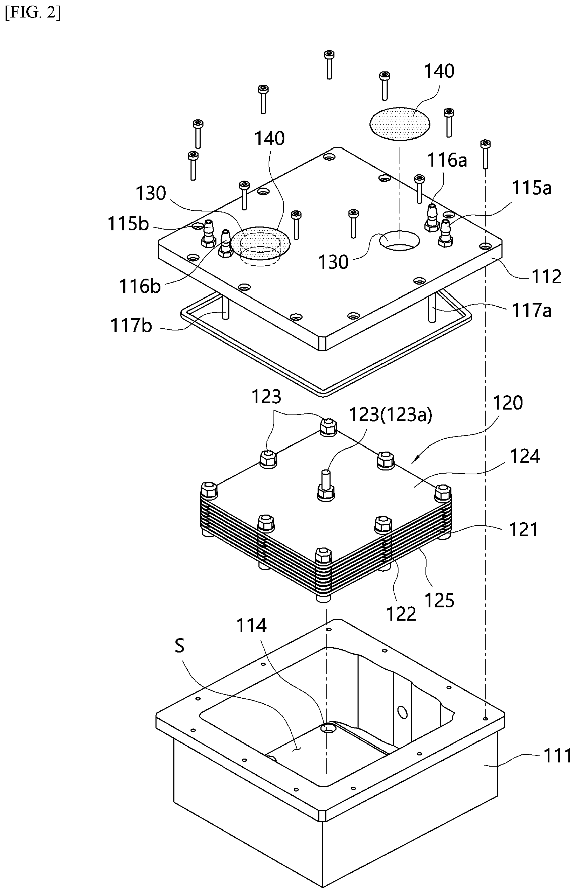

For example, as shown in FIG. 2, the housing 110 may include a housing body 111 in a box shape having a culture space S with an open upper surface and a cover 112 covering the open upper portion of the housing body 111.

In this case, a seeding port 113 for seeding the suspended cells into the culture space S by introducing a medium including suspended cells from the outside into the culture space S may be formed on one side of the housing body 111.

Through this, the culture space S may be filled with a medium supplied from the outside through the seeding port 113, and the suspended cells that have completed cell culture in the culture space S may be discharged to the outside through the seeding port 113 together with the medium.

That is, the seeding port 113 may also serve as a harvesting port that discharges the medium including the suspended cells from the culture space S to the outside so that the suspended cells that have completed culture may be harvested with a medium supply port for supplying a medium to the culture space S from the outside to seed suspended cells in the culture space S.

However, the present invention is not limited to this, a seeding port for seeding the suspended cells in the culture space S and a harvesting port that discharges the medium including the suspended cells from the culture space S to the outside so that suspended cells that have completed culture may be harvested may be formed separately on one side of the housing body 111.

At this time, the culture space S may be divided into a medium storage space S1 and a gas storage space S2 through the medium supplied from the outside through the seeding port 113 and stored in a certain amount internally, as described above.

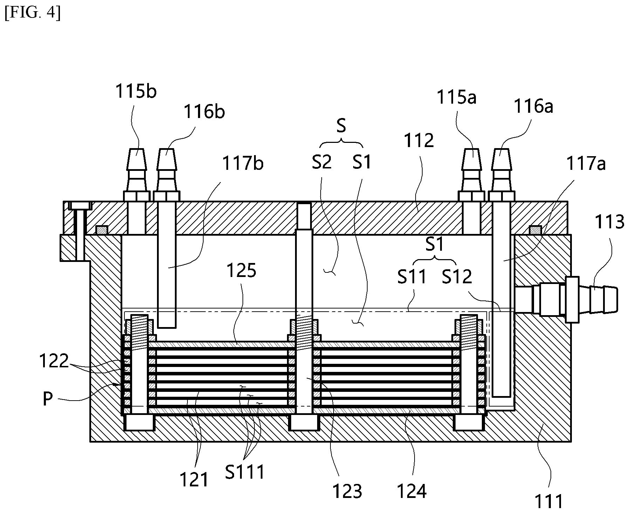

That is, as shown in FIG. 4, the culture space S may be divided into a medium storage space S1 where the medium is stored through the level of the medium filled inside, and a gas storage space S2 where gas is stored.

In this case, the support assembly 120 may be disposed to be submerged in the medium storage space S1, and the medium storage space S1 may be divided into a plurality of cell growth spaces S111 through a plurality of supports 121 disposed in multiple stages at intervals as described above. Accordingly, in the bioreactor for suspended cells 100 according to one embodiment of the present invention, since one medium storage space S1 filled with the medium may be divided into a plurality of cell growth spaces S111 where cells may grow, the frequency of contact between the suspended cells floating in the medium and the support 121 may be increased.

Through this, the bioreactor for suspended cells 100 according to one embodiment of the present invention may increase the frequency of contact between suspended cells and the support 121 even when a support having the same total area in a confined cell growth space S111 defined by two supports 121 is used.

That is, in the bioreactor for suspended cells 100 according to one embodiment of the present invention, since the cells may be suspended in a limited volume of the cell growth space S111 through two supports 121 even when the number of suspended cells included in the medium per unit area or per unit volume is the same compared to a conventional backpacktype bioreactor, the suspended cells included in the medium per unit area or per unit volume may be in more frequent contact with one surface of each of two supports 121 disposed at the upper and lower portions.

Accordingly, since the frequency of contact between the suspended cells included in the medium and the support 121 may be increased, in the bioreactor for suspended cells 100 according to one embodiment of the present invention, cells may grow more smoothly through frequent cell-to-cell interactions.

The support assembly 120 may include a plurality of supports 121 for transmitting a signal to the suspended cells in the medium when coming into contact with the suspended cells. In this case, as described above, in the support assembly 120, the plurality of supports 121 may be disposed in multiple stages at intervals from each other, thereby dividing one medium storage space S 1 into a plurality of cell growth spaces S111.

Accordingly, as described above, as the process of floating in the medium, coming into contact with the support 121, and then falling is repeated, suspended cells included in the medium may grow by receiving signals from the supports 136 and 136' and receiving nutrients from the medium.

To this end, the support assembly 120 may smoothly culture a large number of cells by increasing the integration of the plurality of supports 121 and may be configured to maintain a plurality of cell growth spaces S111 separated from each other while increasing assemblability.

For example, the support assembly 120 may be configured as a stacked structure in which a plurality of supports 121 are disposed in parallel and spaced apart along the height direction of the housing 110.

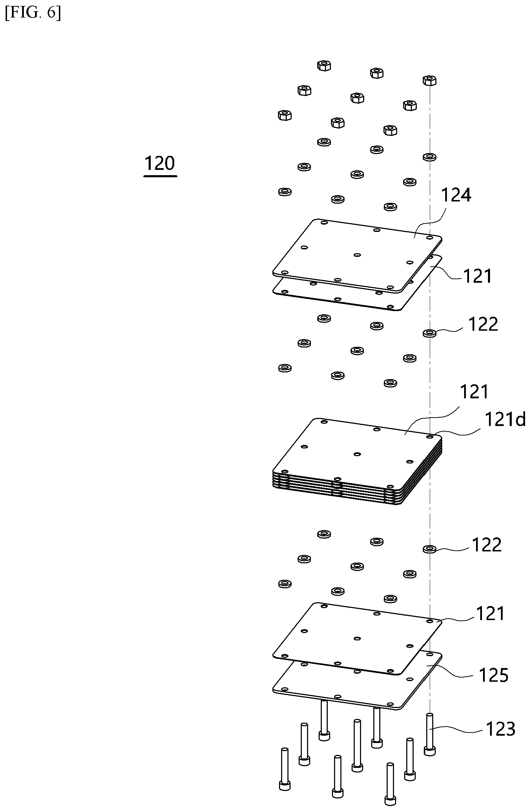

As a specific example, as shown in FIG. 6, the support assembly 120 may include a plurality of fastening bars 123 having a predetermined length and a plurality of spacing members 122 fastened to the fastening bars 123, and the plurality of supports 121 may each be fitted onto the fastening bar 123.

Herein, the spacing member 122 may be provided as a ring-shaped member so that it may be individually fastened to one fastening bar 123, as shown in FIG. 6.

In this case, the plurality of fastening bars 123 may be spaced apart from each other at predetermined intervals, and the plurality of fastening bars 123 may be fixed to each of a plate-shaped upper plate 124 and lower plate 125 of which both ends have a predetermined area.

Accordingly, the plurality of fastening bars 123, both ends of which are respectively fixed to the upper plate 124 and the lower plate 125, may be maintained at a distance from each other, the plurality of supports 121 may be disposed in multiple stages so that one surfaces face each other between the upper plate 124 and the lower plate 125, and the plurality of supports 121 may each be fastened to the fastening bars 123 through a plurality of fastening holes 121d formed through positions corresponding to the plurality of fastening bars 123.

In addition, the plurality of spacing members 122 may each be inserted onto the plurality of fastening bars 123 like the supports 121, and the plurality of spacing members 122 and the plurality of supports 121 may be alternately fastened to each fastening bar 123.

Accordingly, each spacing member 122 may be disposed between two supports 121 disposed along the height direction of the housing 110, two neighboring supports 121 may maintain a distance from each other through the spacing member 122, and the upper plate 124, the lower plate 125, the plurality of supports 121, and the spacing members 122 may be integrated through the plurality of fastening bars 123.

Through this, a plurality of cell growth spaces S111 divided by two supports 121 arranged along the height direction of the housing 110 while having the same height as the height of the spacing member 122 may be formed between the plurality of supports 121.

However, the support assembly 120 is not limited to this, and various known methods may be applied as long as the plurality of supports 121 are arranged parallel to each other in one direction and are spaced apart from each other at regular intervals.

At this time, at least one fastening bar 123a among the plurality of fastening bars 123 is disposed in the culture space S so that the lower end comes into contact with a bottom surface of the culture space S and the upper end comes into contact with an inner surface of the cover 112.

For example, among the plurality of fastening bars 123, the fastening bar 123a fastened to the central portion of the support assembly 120 may be disposed in the culture space S so that both ends come into contact with the bottom surface of the culture space S and the inner surface of the cover 112.

In this case, fixing grooves 114a and 114b that are recessed to a certain depth may be respectively formed in an inner surface of the housing body 111 and the inner surface of the cover 112 so that both ends of the fastening bar 123a are inserted and fixed to a certain depth.

Through this, when the culture space S is divided into a medium storage space S1 filled with a medium through the level of the medium filled inside and a gas storage space S2 filled with gas and the support assembly 120 is disposed to be submerged in the medium storage space S1, even when buoyancy occurs on the side of the support assembly 120 disposed to be submerged in the medium storage space S1, the support assembly 120 can be prevented from floating from the medium storage space S1 to the gas storage space S2 by buoyancy through the fastening bar 123a.

Accordingly, since each cell growth space S111 defined through two supports 121 in the support assembly 120 may always be maintained in a state filled with a medium, the suspended cells included in the medium may be smoothly cultured in the cell growth space S111.

Herein, the fixing groove 114a formed in the inner surface of the housing body 111 may be provided to correspond one to one with the plurality of fastening bars 123 at positions corresponding to the plurality of fastening bars 123.

Meanwhile, the surface of the support 121 may have a surface coated with a peptide having cell proliferation properties.

That is, the surface of the support 121 may be coated with a peptide motif that has cell proliferation properties.

In addition, the support 121 may be provided in a plate shape with a predetermined area to expand the area that may be contacted by suspended cells included in the medium.

Accordingly, each cell growth space S111 divided by a plurality of supports 121 disposed in multiple stages at intervals along the height direction of the housing 110 may be filled with a medium including the suspended cells.

Accordingly, the bioreactor for suspended cells 100 according to one embodiment of the present invention may increase the degree of integration of the support 121 for cell culture, and since plate-shaped supports 121 are disposed on the upper and lower sides of each of the plurality of cell growth spaces S111, suspended cells floating in each cell growth space S111 may be in more frequent contact with the surface of the support 121.

Through this, the bioreactor for suspended cells 100 according to one embodiment of the present invention may culture a larger amount of cells through one culture, and since suspended cells floating within the cell growth space S111 may more smoothly contact the surface of the support 121, the suspended cells may grow more smoothly in each separate cell growth space S111, and cell-to-cell interactions and peptide signals can occur more frequently, which may promote the growth of suspended cells.

In addition, since the bioreactor for suspended cells 100 according to one embodiment of the present invention may form a plurality of cell growth spaces S111 through a plurality of supports 121 disposed in multiple stages in one medium storage space S1, the overall size of the housing 110 may be reduced while it is still possible to culture a large number of cells.

To this end, the support 121 may include a nanofiber membrane 121a in which nanofibers are formed into a three-dimensional network structure through electrospinning, and the nanofiber membrane 121a may be provided in pairs to form both surfaces of the support 121.

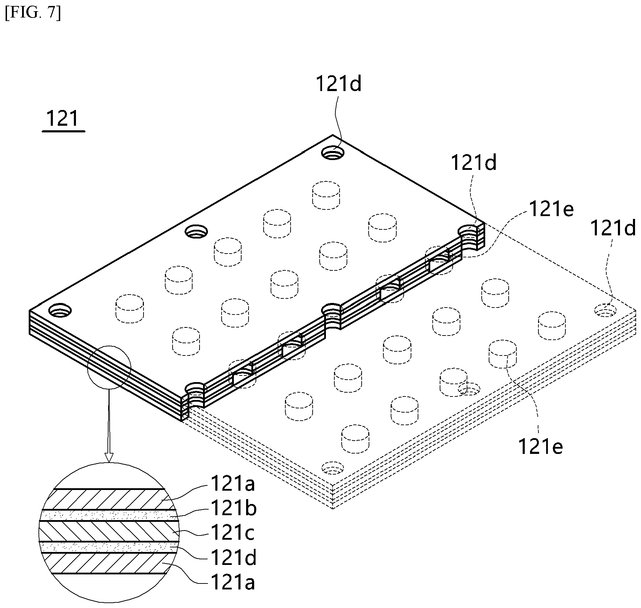

As an example, the support 121 may have a five-layer structure in which a pair of nanofiber membranes 121a are attached to both sides of a support member 121c via an adhesive layer 121b, as shown in FIG. 7.

Herein, the support member 121c may be a plate-shaped film member and may support one surface of the nanofiber membrane 121a. Through this, even when the nanofiber membrane 121a has flexibility and is formed in a plate shape, it can be supported through the support member 121c, thereby preventing bending or sagging.

Accordingly, the support 121 disposed in the medium storage space S1 may be maintained in an unfolded state, so that cells may be cultured smoothly. In addition, the nanofiber membrane 121a forming the surface of the support 121 may be a plate-shaped nanofiber membrane coated with a peptide motif that has cell proliferation properties so that the surface of the support 121 has cell proliferation properties, as described above.

At this time, in the bioreactor for suspended cells 100 according to one embodiment of the present invention, even when the total number of supports 121 arranged in multiple stages to define a plurality of cell growth spaces S111 in the medium storage space S1 increases, the gas required for cell culture may be smoothly supplied to the medium filled in each culture space S defined through the two supports 121.

To this end, as shown in FIG. 7, the support 121 may include a plurality of through holes 121e.

That is, the plurality of through holes 121e may serve as a passage through which gas introduced into the gas storage space S2 from the outside through the gas inlet 130 moves downward from the gas storage space S2 through the support 121. Through this, the plurality of through holes 121e may improve gas flowability.

To this end, the plurality of through holes 121e may be formed in a portion of the support 121 that does not allow gas to pass through. For example, the plurality of through holes 121e may be formed in the support 121 to pass through the adhesive layer 121b and the support member 121c.

Accordingly, the gas introduced into the gas storage space S2 through the gas inlet 130 may smoothly move to the lower side through the plurality of through holes 121e formed in the support 121.

Accordingly, the medium filled in each cell growth space S111 may smoothly receive gas from the gas storage space S2 through the plurality of through holes 121e regardless of location.

At this time, as shown in FIG. 4, the medium storage space S1 may include a first space S11 in which the support assembly 120 is disposed and a second space S12 formed between the support assembly 120 and an inner surface of the housing 110 where the seeding port 113 is formed.

In this case, the first space S11 may be a space in which the support assembly 120 is disposed, and the first space S11 may be divided into a plurality of cell growth spaces S111 through the plurality of supports 121.

In addition, the second space S12 may be a space in which the support assembly 120 is not disposed, and the second space S12 may be a space where the medium supplied from the outside through the seeding port 113 stays before flowing into each cell growth space S 111.

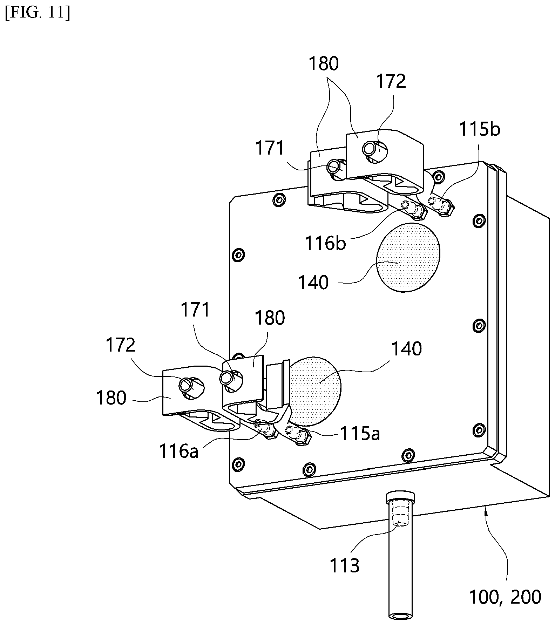

For example, as shown in FIG. 11, after arranging the bioreactor for suspended cells 100 so that the seeding port 113 is disposed at the lower portion, when a medium is supplied from the outside through the seeding port 113, the medium supplied from the outside may fill a portion of the second space S12 and the gas storage space S2.

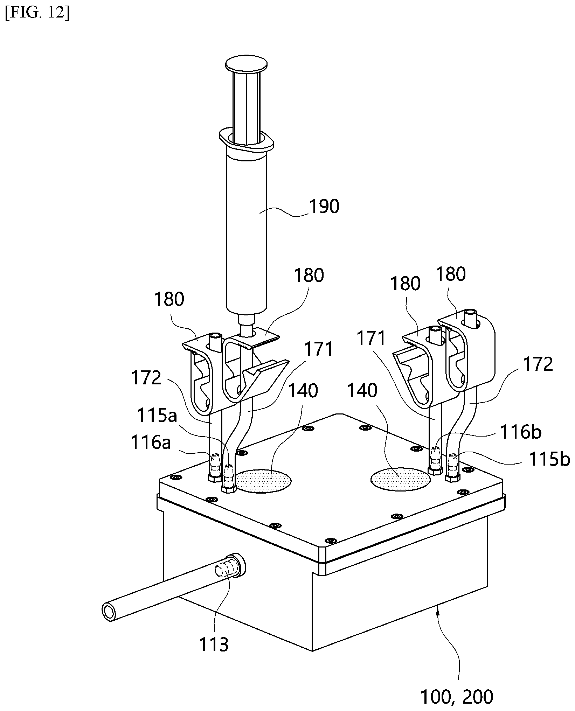

Then, when the bioreactor for suspended cells 100 according to one embodiment of the present invention is changed to the state shown in FIG. 12, the medium that fills some of the second space S12 and the gas storage space S2 may move to each cell growth space S111, forming the medium storage space S1 and the gas storage space S2 described above.

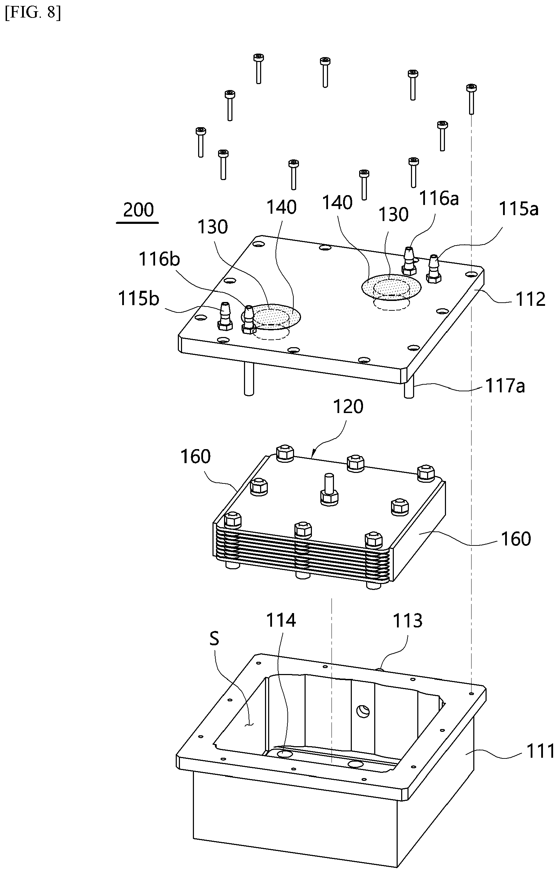

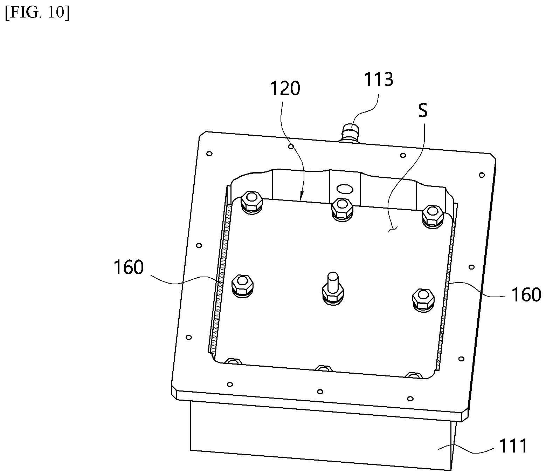

Meanwhile, as shown in FIGS. 8 and 10, the bioreactor for suspended cells 100 according to one embodiment of the present invention may further include at least one blocking member 160 fastened to a side of the support assembly 120 to cover the open side of each cell growth space S111 separated by two supports 121.

For example, the blocking member 160 may be fastened to the side of the support assembly 120 to cover both left and right sides of the support assembly 120 based on FIGS. 8 and 10.

Herein, the blocking member 160 may include a plate-shaped body 161 having a predetermined area and a plurality of fastening grooves 162 formed to be recessed inward at intervals along the height direction on one surface of the body 161, and the end of the support 121 may be inserted into the fastening groove 162.

However, the shape of the blocking member 160 is not limited to this, and as long as the open side of the cell growth space S111 may be covered by covering the side of the support assembly 120, the shape may be changed to various shapes depending on design conditions.

Accordingly, in the bioreactor for suspended cells 100 according to one embodiment of the present invention, when the support assembly 120 is disposed in the culture space S and the culture space S is filled with a certain amount of medium and divided into a medium storage space S1 and a gas storage space S2, each cell growth space S111 divided by two supports 121 in the medium storage space S1 can be sealed on both sides through the blocking member 160.

Accordingly, each cell growth space S111 can be defined through the blocking member 160 along with the two supports 121, so that it may be more clearly distinguished from each other.

The gas inlet 130 may be formed through the housing 110 by a predetermined area to allow gas required for culturing cells to flow into the culture space S from the outside.

Such a gas inlet 130 may be formed in the housing 110 to communicate with the gas storage space S2.

That is, the bioreactor for suspended cells 100 according to one embodiment of the present invention may include at least one gas inlet 130 that communicates with the gas storage space S2 to allow gas to flow into the medium filled in the medium storage space S1.

For example, the gas inlet 130 may be formed to pass through the cover 112.

Accordingly, when culturing cells using the bioreactor for suspended cells 100 according to one embodiment of the present invention, when the bioreactor for suspended cells 100 is disposed inside a chamber such as an incubator, gas present inside the incubator may flow into the gas storage space S2 through the gas inlet 130, and the gas flowing into the gas storage space S2 may be supplied to the medium filled in the medium storage space S1.

Herein, the incubator may be a space that provides a culture environment for suspended cells included in the medium. For example, the incubator may be a chamber, and the inside of the chamber may be an environment in which the temperature and concentration of carbon dioxide are kept constant.

In addition, the gas may be carbon dioxide gas, but is not limited thereto and may be appropriately changed depending on the cells to be cultured.

In this case, the incubator may include an air conditioning system to maintain the internal temperature at a constant temperature, and may include a gas supply unit (not shown) for stably supplying gas necessary for culturing cells into the incubator and maintaining the gas concentration inside the incubator at a certain level.

Through this, in the bioreactor for suspended cells 100 disposed inside the incubator, gas present inside the incubator may flow into the gas storage space S2 through the gas inlet 130, the gas introduced into the gas storage space S2 may be dissolved in the medium filled in the medium storage space S1, and the medium may be maintained at an appropriate pH required for cell culture through the dissolution of the gas.

Accordingly, the medium filled in the medium storage space S1 may be maintained at an appropriate pH required for cell culture, so that the suspended cells included in the medium may be cultured smoothly.

Meanwhile, the gas inlet 130 may be covered with a plate-shaped porous member 140 having a predetermined area. That is, the porous member 140 may be attached to one surface of the cover 112 to cover the gas inlet 130 formed in the cover 112.

This porous member 140 may block foreign substances and liquids from passing through, while allowing gases such as carbon dioxide to pass through. Through this, other foreign substances may not enter the gas storage space S2, and the medium filled in the medium storage space S1 may be smoothly supplied with the gas necessary for culturing cells from the gas storage space S2.

Accordingly, even when gas flows into the gas storage space S2 from the outside through the gas inlet 130, the medium filled in the medium storage space S1 may not be contaminated by other foreign substances.

For example, the porous member 140 may be a water-repellent nanofiber membrane. However, the material of the porous member 140 is not limited to this, and any material that allows gaseous fluid to pass through while blocking solids and liquid fluids from passing through may be used without limitation.

Meanwhile, the bioreactor for suspended cells 100 according to one embodiment of the present invention may further include one or more vent holes 115a and 115b formed in the housing 110, and one or more ports 116a and 116b formed in the housing 110 to supply a medium to the culture space S or discharge the medium present in the culture space S to the outside.

For example, the vent holes 115a and 115b may be formed in the housing 110 to communicate with the gas storage space S2, and each of the vent holes 115a and 115b and the ports 116a and 116b may be provided as one, but may also be provided in plural numbers.

In addition, each of the vent holes 115a and 115b and the ports 116a and 116b may be connected to separate connecting tubes 171 and 172, respectively, and separate opening and closing unit 180 may be provided on the connecting tubes 171 and 172 to allow or block the movement of fluid, respectively. For example, the opening and closing unit 180 may be a known clip-type opening and closing valve.

In this case, the bioreactor for suspended cells 100 according to one embodiment of the present invention may further include extension tubes 117a and 117b having a predetermined length connected to the ports 116a and 116b in the culture space S, and the lower end of the extension tubes 117a and 117b may be disposed in the culture space S to be located at a lower position than the support located at the top among the plurality of supports 121 constituting the support assembly 120.

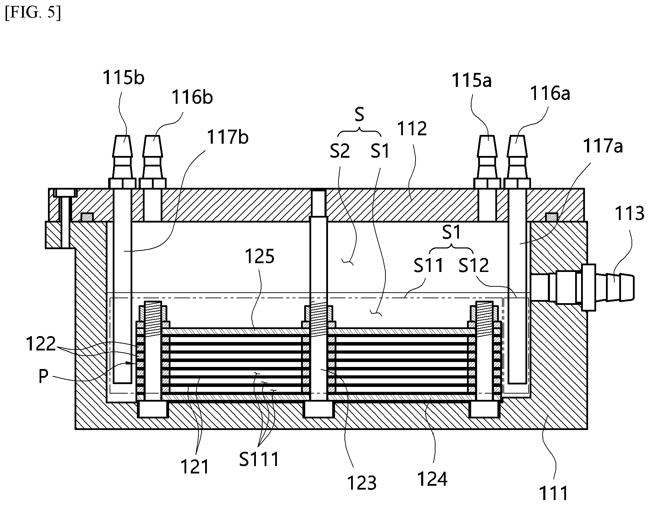

That is, the extension tubes 117a and 117b may be disposed so that their lower ends are submerged in the medium storage space S1, as shown in FIGS. 4 and 5, and preferably, the extension tubes 117a and 117b may be disposed in the culture space S so that their lower ends are located close to a bottom surface of the housing body 111 forming the bottom surface of the culture space S.

Herein, when a plurality of ports 116a and 116b are provided, a plurality of extension tubes 117a and 117b may be provided in a one-to-one connection with the plurality of ports 116a and 116b.

In this case, as shown in FIG. 4, some of the extension tubes 117a among the plurality of extension tubes 117a and 117b are disposed in the culture space S so that their lower ends are disposed close to the bottom surface of the housing body 111 forming the bottom surface of the culture space S, and the remaining extension tubes 117b may be disposed in the culture space S so that their lower ends are spaced a certain distance from the bottom surface of the housing body 111 forming the bottom surface of the culture space S. Alternatively, as shown in FIG. 5, each of the plurality of extension tubes 117a and 117b may be disposed in the culture space S so that their lower end is located close to the bottom surface of the housing body 111 forming the bottom surface of the culture space S.

Herein, the ports 116a and 116b may serve to additionally supply a new medium required for cell culture in addition to the existing medium filled in the culture space S, and may serve to replace part or all of the existing medium filled in the culture space S.

In this case, the vent holes 115a and 115b may control the internal pressure of the culture space S so that the gas in the gas storage space S2 can be discharged to the outside in the process of supplying a medium to the culture space S or discharging the medium filled in the culture space S to the outside.

Accordingly, even when a new medium for addition or replacement is supplied to the culture space S from the outside through the ports 116a and 116b, it is possible to prevent the generation of bubbles in the culture space S while the medium is input through the ports 116a and 116b by controlling the internal pressure through the vent holes 115a and 115b.

As a non-limiting example, when the port 116a serves to additionally supply a new medium required for cell culture in addition to the existing medium filled in the culture space S, as shown in FIG. 12, the port 116a can be connected to a medium injection unit 190 through the connecting tube 171, and the medium supplied from the medium injection unit 190 may move to the medium storage space (S1) through the connecting tube 171, the port 116a, and the extension tube 117a.

Accordingly, since the medium supplied from the medium injection unit 190 can be directly introduced into the medium storage space S1 without being supplied to the gas storage space S2 through the extension tube 117a whose one end is submerged in the medium storage space S1, the medium may minimize the flow of the medium due to the inflow pressure without interfering with the growth of cells.

Consequently, the medium added to the medium storage space S1 through the port 116a may be mixed smoothly with the existing medium present in the medium storage space S1 so that the nutrients included in the medium may be evenly distributed per unit volume.

Through this, since the cell culture medium may be easily added to the medium storage space S1 through the port 116a, and the existing medium and new medium may be smoothly mixed, the bioreactor for suspended cells 100 according to one embodiment of the present invention may promote the growth of suspended cells.

In this case, some of the gas storage space S2 may be converted into the medium storage space S1 by the volume of the medium added through the ports 116a and 116b.

As other example, when the ports 116a and 116b serve to replace part or all of the medium filled in the culture space S, the ports 116a and 116b may be composed of a plurality of ports 116a and 116b. In this case, some of the ports 116a of the plurality of ports 116a and 116b may serve as a supply port for supplying a medium, and the remaining ports 116b may serve as a discharge port for discharging the medium.

Through this, since all or part of the existing medium filled in the culture space S may be easily replaced through the plurality of ports 116a and 116b, the bioreactor for suspended cells 100 according to one embodiment of the present invention may more smoothly supply the nutrients necessary for the growth of suspended cells.

That is, when all or part of the existing medium filled in the culture space S is replaced with a new medium, the concentration of nutrients included in the medium per unit volume may be higher compared to adding a new medium to the existing medium.

Accordingly, when all or part of the existing medium is replaced with a new medium, since the suspended cells cultured in the cell growth space S111 may more smoothly receive nutrients included in the medium, the growth of the suspended cells may be promoted.

Herein, the plurality of ports 116a and 116b may be divided into supply ports and discharge ports, but may also serve as the supply ports and discharge ports.

That is, the plurality of ports 116a and 116b may serve as supply ports that supply a new medium to the culture space S and then may be converted to a role as discharge ports that discharge the medium filled in the culture space S to the outside, or may serve as discharge ports that discharge the medium filled in the culture space S to the outside and then may be converted to a role as supply ports that supply a new medium to the culture space S.

Although one embodiment of the present invention has been described above, the spirit of the present invention is not limited to the embodiment presented herein, and those skilled in the art and having an understanding of the scope of the invention will readily suggest other embodiments by altering, deleting, adding, etc., components within the same spirit, which are also intended to fall within the spirit of this invention.

## Claims

1. A bioreactor for suspended cells comprising:
a housing formed in a box shape having a culture space with a predetermined volume and including a seeding port for seeding suspended cells into the culture space through a medium containing the suspended cells on one side;
a support assembly formed of a plate-shaped member having a predetermined area and including a plurality of supports disposed in multiple stages at intervals along one direction of the housing;
a gas inlet formed through the housing by a predetermined area to allow gas to flow into the culture space from the outside; and
a porous member covering the gas inlet to allow the gas to enter the culture space from the outside while preventing the medium from leaking to the outside,
wherein the culture space includes a medium storage space filled with a certain amount of medium so that all of the plurality of supports are submerged and a gas storage space located above the medium storage space and filled with gas, and
the medium storage space includes a plurality of cell growth spaces separated from each other by the plurality of supports disposed in multiple stages at intervals.

2. The bioreactor for suspended cells of claim 1, wherein the support includes a plate-shaped support member having a predetermined area, and a pair of nanofiber membranes respectively attached to both sides of the support member via an adhesive layer, and
the nanofiber membrane is a motif-coated plate-shaped nanofiber membrane.

3. The bioreactor for suspended cells of claim 2, wherein the support includes a plurality of through holes formed through the support member to allow gas introduced into the gas storage space from the outside to pass smoothly.

4. The bioreactor for suspended cells of claim 1, wherein the support assembly includes:
upper and lower plates with a predetermined area;
a plurality of supports disposed between the upper and lower plates so that one surfaces face each other;
a spacing member disposed between two supports facing each other so as to separate the two supports; and
a plurality of fastening bars that fasten and integrate the upper plate, the lower plate, the plurality of supports, and the spacing member.

5. The bioreactor for suspended cells of claim 4, wherein the housing includes a housing body in a box shape having a culture space with an open upper portion and a cover covering the open upper portion of the housing body, and
at least one fastening bar among the plurality of fastening bars is disposed in the culture space so that a lower end thereof comes into contact with a bottom surface of the culture space and an upper end thereof comes into contact with an inner surface of the cover to prevent the support assembly from floating in the culture space.

6. The bioreactor for suspended cells of claim 5, wherein fixing grooves that are recessed to a certain depth are respectively formed in an inner surface of the housing body and the inner surface of the cover so that both ends of the fastening bar are inserted and fixed to a certain depth.

7. The bioreactor for suspended cells of claim 1, comprising at least one blocking member fastened to a side of the support assembly to cover an open side of the cell growth space.

8. The bioreactor for suspended cells of claim 1, wherein the support assembly is disposed in the medium storage space to be spaced a certain distance from an inner surface of the housing where the seeding port is formed,
the medium storage space includes a first space in which the support assembly is disposed and a second space formed between the support assembly and the inner surface of the housing where the seeding port is formed, and
the first space is divided into the plurality of cell growth spaces through the plurality of supports.

9. The bioreactor for suspended cells of claim 1, wherein the porous member is a water-repellent membrane.

10. The bioreactor for suspended cells of claim 1, further comprising a vent hole formed in the housing to communicate with the gas storage space and a port formed in the housing to supply a medium to the culture space or discharge the medium present in the culture space to the outside.

11. The bioreactor for suspended cells of claim 10, further comprising an extension tube connected to the port in the culture space and having a predetermined length,
wherein a lower end of the extension tube is disposed in the culture space to be located at a lower position than the support located at the top among the plurality of supports constituting the support assembly.
